(19) Europäisches Patentamt
European Patent Office
Office européen des brevets

(11) **EP 4 124 858 A1**

(12) **EUROPEAN PATENT APPLICATION**

(43) Date of publication:
**01.02.2023 Bulletin 2023/05**

(21) Application number: **21187850.9**

(22) Date of filing: **27.07.2021**

(51) International Patent Classification (IPC):
**G01N 27/48** (2006.01)    **G01N 33/28** (2006.01)

(52) Cooperative Patent Classification (CPC):
**G01N 27/48; G01N 33/2888**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Metrohm AG**
**9100 Herisau (CH)**

(72) Inventor: **Arumugam, Sivanesan**
**Carlingford, 2118 (AU)**

(74) Representative: **Hepp Wenger Ryffel AG**
**Friedtalweg 5**
**9500 Wil (CH)**

(54) **METHOD FOR THE SIMULTANEOUS DETERMINATION OF ANTIOXIDANTS AND ANTI-WEAR ADDITIVES**

(57)    The present invention refers to a method for simultaneous determination of antioxidants and/or anti-wear additives in lubricants, in particular amine, zinc dithiophosphate (ZDDP) and phenol. The method comprises the steps of:
a) providing an electrolyte solution with a pH between 3 and 10, preferably pH 6.5,
b) combining the electrolyte solution, a sand composition and a lubricant to obtain a mixture,
c) extracting the mixture, preferably by vortexing, to obtain an extracted solution,
d) measuring at least an aliquot of the extracted solution by means of pulse voltammetry, preferably differential pulse voltammetry, and determining the amounts of antioxidants.

Fig. 1

EP 4 124 858 A1

**Description**

The invention relates to a method for determining antioxidants and/or anti-wear additives with a pulse voltammetry technique, in particular differential pulse voltammetry. The invention also refers to an analysis kit for said method.

Antioxidants and anti-wear additives are used to prolong the life of a lubricant by increasing the oxidative resistance of the base oil. Antioxidants allow lubricants to operate at higher temperatures than would otherwise be possible without them.

Many synthetic lubricants, especially hydrocarbon-based lubricating oils, are susceptible to degradation by oxygen. This oxidation process, which is initiated by the formation of reactive free radicals and peroxides, is the major cause of oil thickening and the formation of sludge and varnish in many applications.

There are two types of antioxidants: primary and secondary antioxidants. Primary antioxidants are typically comprised of aromatic amines and hindered phenolics. Secondary antioxidants are typically comprised of phosphites and certain sulfur-containing compounds, such as thioethers and thioesters. Each type of antioxidant performs a different function to inhibit oxidation.

The oxidation process begins with the initiation phase, when free radicals are formed. Primary antioxidants are "radical scavengers" that react quickly with the free radicals during the propagation phase, slowing down the degradation process by forming new radicals that are more stable. Secondary antioxidants react with peroxides which are often present as the lubricating oil reacts with oxygen. These antioxidants are responsible for breaking the cycle and preventing branching and further propagation. Most often, grease formulators will use a combination of primary and secondary antioxidants to maximize the protection of the oil against oxidative degradation.

However, getting the right combination of antioxidants is critical to the grease's performance in the conditions for a respective application. Thus, there is a need for a method that can qualitatively and quantitatively determine antioxidants and anti-wear additives in a fresh or used lubricant.

The currently known ASTM method (D6810, D6971, D7527, and D7590) recommends four different types of electrolyte solutions depending on the oil type: turbine, hydraulic, jet engine & Diesel. The method can only determine two additives (amine and phenol) and for the presence of hindered phenol even needs two separated extractions steps. This makes the current method rather time consuming and expensive. Further, two analysis kit are needed to analyse one oil sample. In addition, the method is not sensitive and if the remaining amine or phenol drops under a concentration of 30 %, the amine or phenol cannot be determined anymore. The method also lacks reproducibility and automation.

It is one objective of the present invention to overcome the drawbacks of the prior art. In particular, it is an objective to provide a method that can determine antioxidants and anti-wear additives simultaneously. Further, it is another objective of the invention to provide an analysis kit for the simultaneous determination of antioxidants and anti-wear in lubricants.

The objectives are achieved by the subject-matter defined in the independent claims. Further embodiments are described in the dependent claims.

It is a first aspect of the invention to provide a method for simultaneous determination of antioxidants in lubricants, in particular amine, zinc dithiophosphate (ZDDP) and phenol. The method comprises the steps of:

    a) providing an electrolyte solution with a pH between 3 and 10, preferably pH 6.5,
    b) combining the electrolyte solution, a sand composition and a lubricant to obtain a mixture,
    c) extracting the mixture, preferably by vortexing, to obtain an extracted solution,
    d) measuring at least an aliquot of the extracted solution by means of pulse voltammetry, preferably differential pulse voltammetry, and determining the amounts of antioxidants.

The method has the advantage that several antioxidants and anti-wear additives can be determined simultaneously without the need of different electrolyte solutions or extraction steps. This method provides a fast, cost-saving and efficient way to determine the antioxidants and anti-wear composition in lubricants.

In step b) only one single electrolyte solution is required. After combination with sand in step b) and after extraction in step c, for example by vortexing, the sand can be allowed to settle down before aliquots of the sample are taken. This avoids a possibility of blocked channels during analysis.

Preferably, the electrolyte solution is a buffered solution, preferably a TRIS-(tris(hydroxymethyl)aminomethane)-salt buffered solution, herein referred to as TRIS electrolyte. In case of a TRIS-salt buffer, perchloric acid ($HClO_4$) in anhydrous acetic acid may be added to obtain a TRIS-salt/$HClO_4$ acetic acid buffer. Other buffer may be BIS TRIS (bis(2-(hydroxyethyl)amino-tris(hydroxymethyl)methane), citrate, acetate or other similar buffers having a hydroxy or amine functionality.

The buffer is particularly advantageous for the voltammetric peak separation of amine, ZDDP and phenol.

Further, the electrolyte solution may comprise a mixture of organic solvents, preferably a methanol/ethanol mixture. Other organic solvents may also be used. Preferably, the organic solvent is chosen from a group consisting of methanol, ethanol, propanol, isopropanol or acetone or a mixture thereof. Mixtures with a 1:1 ratio are preferred, but others may also be suitable. Particularly preferred is a methanol/ethanol mixture with a 1:1 (V/V) ratio.

The use of organic solvents or mixed organic solvents has the advantage that all the different additives in the lubricant sample can be extracted efficiently. Best results were obtained with a 1:1 methanol/ethanol mixture.

Advantageously, the sand is white quartz sand. Typically, white quartz sand consists of at least 95 % $SiO_2$ and less than 0.6 % iron oxide and comprises less impurities than regular sand.

The sand composition can comprise sand of at least two different meshes, preferably two different meshes in the range of 50 to 230. For example, the sand composition can comprise sand with mesh of 50-70 and sand of another mesh of 230.

Sand of different mesh allows for absorption of fine and larger oil droplets and thus avoids film formation on the extract. Film formation is disadvantageous since it may lead to fouling of the electrode surface.

The lubricant used in the method according to the invention is preferably an oil or mixture of oils, preferably hydraulic oil, turbine oil, engine oil or mixtures thereof.

For example, different oils may contain different amounts of additives. Sometimes the oil in an automotive engine or the like is refilled without the old oil being completely consumed. If not the exact same oil is added but a different oil, different additives can be present in the oil, resulting in a mixture of oils.

Further, step d) is preferably preceded by a blank measurement of the electrolyte solution. A blank solution contains little to no analyte of interest. Blank measurements avoid backgrounds interference and provide more precise results.

More preferably, a standard can additionally be used in order to determine the correct concentration of the antioxidants and anti-wear components. A standard solution comprises the respective components in known concentrations.

The following equation can be used in order to determine the concentration of the antioxidants and anti-wear additives:

$$remaining\ \%\ of\ antioxidants = \frac{sample\ reading - blank\ reading}{standard\ reading - blank\ reading}\ x\ 100\ \%$$

With a blank and standard solution, the concentration may be determined by a software (e.g. viva software, Metrohm AG, Switzerland) and can be calculated automatically.

Step d) can be preceded with an electrode cleaning cycle to optimise the cleaning of the electrode and to prevent premature wear of the electrode.

Potassium hydroxide (KOH) can be added to the electrolyte solution in order to determine hindered phenol. Preferably, potassium hydroxide is added to the TRIS electrolyte and added to the lubricant sample after extraction of antioxidants in step c).

Advantageously, the potassium hydroxide is a 5 M KOH electrolyte solution.

This allows for a simple determination of hindered phenol without the need of an additional time-consuming method by simply adding one step to said method.

The pulse voltammetry measurement of step d) can be performed with the following parameters:

- a starting potential in the range of -0.2 to 0 V,
- an end potential in the range of 1.3 to 1.5 V,
- a potential step in the range of 0.01 to 0.05 V, preferably 0.012 V,
- a potential step time in the range of 0.05 s to 1 s, preferably 0.1 s,
- a sweep rate in the range of 0.01 to 0.8 V/s, preferably 0.12 V/s,
- a pulse amplitude in the range of 0.001 V to 0.5 V, preferably 0.05 V,
- a pulse time in the range of 0.001 to 0.1 s, preferably 0.04 s,
- a measuring time of 0.02 s, and
- a sweep duration of 4 to 13 s, preferably 10.83 s.

The pulse voltammetry measurement can be any pulse voltammetry: normal pulse, reverse normal pulse and differential pulse. Differential pulse voltammetry is preferred.

A second aspect of the invention refers to an analysis kit for the simultaneous determination of antioxidants and/or anti-wear additives in lubricants in a method as previously described. The kit comprises a sand composition and an electrolyte solution, preferably a sand and electrolyte solution as previously described.

In particular, the kit can have an electrolyte solution with a pH in the range of 3 to 10, preferably 6.5.

Further, the kit can comprise a sand composition with sand of at least two different meshes, preferably in the range of 50 to 230.

Even further, the kit can comprise instructions for use.

The invention is described in more detail by means of the following examples, which are not to be understood in a restrictive manner.

EXAMPLES

1. Reagents

The following reagents were used for the examples:

Type 1 pure water, absolute ethanol (CAS 64-17-5), 99.9 % methanol (CAS 67-56-1), Tris(hydroxymethyl)aminomethane (CAS 67-56-1), sand (Mesh 50-70, CAS 14808-60-7), sand, white quartz > 230 mesh (CAS 14808-60-7), 0.1 M $HClO_4$ in anhydrous acetic acid, potassium hydroxide (CAS 1310-58-3).

2. Preparation of TRIS electrolyte:

TRIS electrolyte was made with 0.05 mol/L TRIS, 100 mL/L $H_2O$, 450 ml/L ethanol and 450 mL/L methanol.

6.057 g of TRIS was dissolved in 100 mL water. The solution was filled up to 1000 mL with 450 mL ethanol and 450 mL methanol. The pH of the solution was adjusted to 6.5 using 0.1 M $HClO_4$ while constant stirring. Around 5 mL 0.1 M $HClO_4$ was consumed to reach pH 6.5.

3. Preparation of KOH

5 M KOH was prepared by dissolving 28.06 g KOH in water and filling up to 100 mL.

4. Extraction method

0.7 g of 50-71 mesh white quartz sand and 0.3 g of 230 mesh white quartz sand were weighted in a 15 mL centrifuge tube. 8 mL of the TRIS electrolyte solution was added. 0.2 mL/0.4 mL of fresh or used oil sample were added. The solution was vortexed for 1 minute in a time-controlled vortex mixer. The sample was given at least one minute in order for the sand particles to settle down before a 5 mL aliquot of the extracted solution was transferred into the measuring vessel.

5. Measurement

5 mL TRIS electrolyte was dosed into a measuring vessel and the blank was recorded. Then the TRIS electrolyte was replaced by 5 mL extraction solution and the calibration was recorded or the oil sample was measured in order to determine the remaining antioxidants/anti-wear additives in the sample.

In case of an oil sample, three measurements per sample for a triple determination were performed and averaged.

Remaining antioxidants, in particular amine, ZDDP and Phenol were calculated using the following equation:

$$remaining \ \% \ of \ antioxidants = \frac{sample \ reading - blank \ reading}{standard \ reading - blank \ reading} \ x \ 100 \ \%$$

If the concentration of amine/ZDDP/phenol antioxidants in the standard is known in percentage or mol/L, the percentage or molar concentration of antioxidants in the sample can be calculated the same way.

As software tool viva (Metrohm AG, Switzerland) was used for sample analysis, the concentration was calculated automatically.

6. Initial cleaning

An initial electrode cleaning cycle can be carried out in the extracted TRIS electrolyte solution just before the analysis. Initial cleaning is performed with the following cyclic voltammetric parameters (table 1):

table 1

| Measuring mode | CV | Potential step | 0.012 V |
|---|---|---|---|
| Stirring rate | 2500 min$^{-1}$ | Potential step time | 0.004 s |
| Pre-treatment | no | Sweep rate | 3 V/s |
| Start potential | 0 V | Preparation cycles | 10 |
| 1$^{st}$ vertex potential | 1.5 V | Measuring cycles | 3 |
| 2$^{nd}$ vertex potential | 0 V | Sweep duration | 13 s |

7. Analysis of oil samples

a) Differential pulse voltammetry parameters for sample analysis

Table 2 shows an example of a set of parameter for differential pulse voltammetry for sample analysis.

table 2

| Measuring mode | DPV | Potential step time | 0.1 s |
|---|---|---|---|
| Stirring rate | 0 min$^{-1}$ | Sweep rate | 0.12 V/s |
| Pre-treatment | no | Pulse amplitude | 0.05 V |
| Start potential | 0 V | Pulse time | 0.04 s |
| End potential | 1.3 V | Measuring time | 0.02 s |
| Potential step | 0.012 V | Sweep duration | 10.83 |

b) Results

The oils and oil mixtures analysed according to the method described above are shown in table 3.

table 3

| Example | Oil type | % amine | % ZDDP | % phenol |
|---|---|---|---|---|
| 1 | Corena 68 | - | 54.4 | 139.7 |
| 2 | Super EX46HN | 46 | - | 12.3 |
| 3 | DTE 846 | 90.3 | - | 65.1 |
| 4 | Titan Utto TO-4 | | 72.3 | |
| 5 | DTE 24 | | 67.1 | |
| 6 | Caltex Delo 400 MGX 15W40 | 44 | 22 | 53 |
| 7 | Mixture of Fuchs Renolin B plus (ZDDP) & Hitachi super Ex 46 (amine & phenol) | 48 | 22 | 48 |

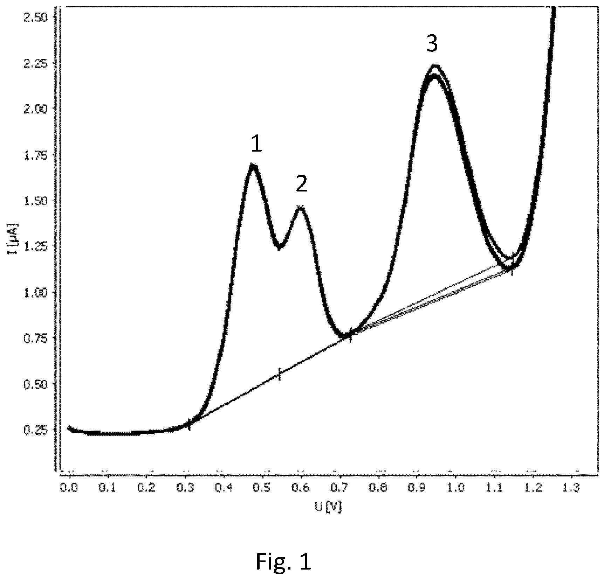

Figure 1 shows the differential pulse voltammogram of example 6 with the potential U[V] on the x-coordinate and the current I[$\mu$A] on the y-coordinate. Peak 1 refers to amine(s), peak 2 refers to ZDDP and peak 3 refers to phenol.

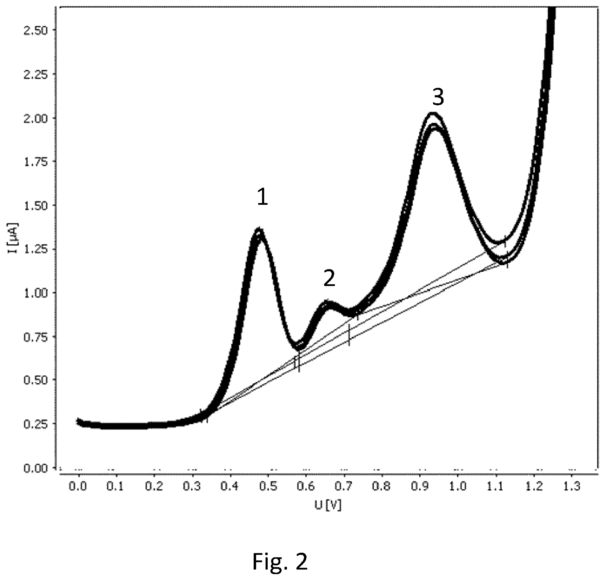

Example 7 contains a mixture of Fuchs Renolin B plus (ZDDP) & Hitachi super Ex 46 (amine & phenol), the respective voltammogram with U[V] on the x-coordinate and I[$\mu$A] on the y-coordinate is shown in figure 2. Peak 1 refers to amine(s), peak 2 refers to ZDDP and peak 3 refers to phenol.

## 8. Determination of hindered phenol

### a) Determination of hindered phenol after analysis of antioxidants

0.3 mL of 5 M KOH was added to 5 mL TRIS electrolyte after extraction and analysis of the antioxidants according to the examples 1 and 5, provided in table 1.

A blank measurement was performed as follows: 5 mL TRIS electrolyte and 0.3 mL of 5 M KOH were dosed into the measuring vessel and the blank was recorded. The TRIS electrolyte and KOH electrolyte were replaced by 5 mL extraction solution and 0.3 mL of 5 M KOH and the remaining antioxidants, in particular the hindered phenol, were determined.

The initial electrode cleaning was conducted as previously described. The sample was analysed by differential pulse voltammetry with the following parameters (table 4):

table 4

| Measuring mode | DPV | Potential step time | 0.1 s |
|---|---|---|---|
| Stirring rate | 0 min$^{-1}$ | Sweep rate | 0.12 V/s |
| Pre-treatment | no | Pulse amplitude | 0.05 V |
| Start potential | 0 V | Pulse time | 0.04 s |
| End potential | 0.5 V | Measuring time | 0.02 s |
| Potential step | 0.012 V | Sweep duration | 4.17 s |

### b) Calculation

Remaining phenol concentration was determined according to the same equation and method as previously described.

### c) Results

The results of the determination of hindered phenol are shown in table 5.

table 5

| Example | Oil type | % ZDDP | % phenol | % hindered phenol |
|---|---|---|---|---|
| 8 | Corena 68 | 54.4 | 139.7 | 91.2 |
| 9 | DTE 24 | 67.1 | - | 78 |

### d) Determination of hindered phenol if only hindered phenol is present

The procedure was performed according the extraction method with the exception that 0.3 mL of KOH was initially added and analysis was carried out with the parameters according to the determination of hindered phenol.

### e) Result

The result of the determination of only hindered phenol is shown in table 6.

table 6

| Example | Oil type | % hindered phenol |
|---|---|---|
| 10 | Thermo 32 ISO 32 | 72.2 |

**Claims**

1. Method for simultaneous determination of antioxidants and/or anti-wear additives in lubricants, in particular amine, zinc dithiophosphate (ZDDP) and phenol, comprising the steps of:

a) providing an electrolyte solution with a pH between 3 and 10, preferably pH 6.5,
b) combining the electrolyte solution, a sand composition and a lubricant to obtain a mixture,
c) extracting the mixture, preferably by vortexing, to obtain an extracted solution,
d) measuring at least an aliquot of the extracted solution by means of pulse voltammetry, preferably differential pulse voltammetry, and determining the amounts of antioxidants.

2. Method according to claim 1, wherein the electrolyte solution is a buffered solution, preferably a TRIS-salt buffered solution.

3. Method according to one of the previous claims, wherein the electrolyte solution comprises a mixture of organic solvents, preferably a methanol/ethanol mixture.

4. Method according to one of the previous claims, wherein the sand is white quartz sand.

5. Method according to one of the previous claims, wherein the sand composition comprises sand of at least two different meshes, preferably two different meshes in the range of 50 to 230.

6. Method according to one of the previous claims, wherein the lubricant is an oil or a mixture of oils, preferably hydraulic oil, turbine oil, engine oil or mixtures thereof.

7. Method according to one of the previous claims, wherein step d) is preceded by a blank measurement of the electrolyte solution.

8. Method according to one of the previous claims wherein step d) is preceded with an electrode cleaning cycle.

9. Method according to one of the previous claims, wherein potassium hydroxide is added to the electrolyte solution to determine hindered phenol.

10. Method according to one of the previous claims, wherein the pulse voltammetry measurement of step d) is performed with:

- a starting potential in the range of -0.2 to 0 V,
- an end potential in the range of 1.3 to 1.5 V,
- a potential step in the range of 0.01 to 0.05 V, preferably 0.012 V,
- a potential step time in the range of 0.05 s to 1 s, preferably 0.1 s,
- a sweep rate in the range of 0.01 to 0.8 V/s, preferably 0.12 V/s,
- a pulse amplitude in the range of 0.001 V to 0.5 V, preferably 0.05 V,
- a pulse time in the range of 0.001 to 0.1 s, preferably 0.04 s,
- a measuring time of 0.02 s, and
- a sweep duration of 4 to 13 s, preferably 10.83 s.

11. Analysis kit for simultaneous determination of antioxidants and/or anti-wear additives in lubricants according to a method as claimed in one of claims 1 to 10, the kit comprising a sand composition and an electrolyte solution.

12. Kit according to claim 11, wherein the electrolyte solution has a pH in the range of 3 to 10, preferably 6.5.

13. Kit according to one of claims 11 or 12, wherein the sand composition comprises sand of at least two different meshes.

14. Kit according to claim 13, wherein the different meshes are in the range of 50 to 230.

15. Kit according to one of claims 11 to 14, the kit further comprising instructions for use.

Fig. 1

Fig. 2

Europäisches
Patentamt
European
Patent Office
Office européen
des brevets

# EUROPEAN SEARCH REPORT

Application Number

EP 21 18 7850

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 103 323 515 B (INSPECTION AND QUARANTINE TECHNOLOGY CT OF NINGBO ENTRY EXIT INSPECTIO) 7 January 2015 (2015-01-07) | 1-6,8, 10-15 | INV. G01N27/48 G01N33/28 |
| Y | * paragraphs [0011] – [0015], [0021] – [0025], [0028], [0092] * | 7,9 | |
| Y | US 4 764 258 A (KAUFFMAN ROBERT E [US]) 16 August 1988 (1988-08-16) * column 2, lines 33-39 * * column 4, lines 35-44 * | 9 | |
| Y | US 5 518 590 A (FANG JIAFU [US]) 21 May 1996 (1996-05-21) * column 5, lines 61-67 * | 7 | |

TECHNICAL FIELDS SEARCHED (IPC)

G01N

The present search report has been drawn up for all claims

| Place of search | Date of completion of the search | Examiner |
|---|---|---|
| Munich | 5 January 2022 | Knoll, Stephan |

EPO FORM 1503 03.82 (P04C01)

## ANNEX TO THE EUROPEAN SEARCH REPORT
## ON EUROPEAN PATENT APPLICATION NO.

EP 21 18 7850

This annex lists the patent family members relating to the patent documents cited in the above-mentioned European search report.
The members are as contained in the European Patent Office EDP file on
The European Patent Office is in no way liable for these particulars which are merely given for the purpose of information.

05-01-2022

| Patent document cited in search report | | Publication date | Patent family member(s) | Publication date |
|---|---|---|---|---|
| CN 103323515 | B | 07-01-2015 | NONE | |
| US 4764258 | A | 16-08-1988 | NONE | |
| US 5518590 | A | 21-05-1996 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- *CHEMICAL ABSTRACTS,* 64-17-5
- *CHEMICAL ABSTRACTS,* 67-56-1
- *CHEMICAL ABSTRACTS,* 14808-60-7
- *CHEMICAL ABSTRACTS,* 1310-58-3